## Europäisches Patentamt
## European Patent Office
## Office européen des brevets

(19)

(11) Numéro de publication: **0 089 859**
**B1**

(12)
# FASCICULE DE BREVET EUROPEEN

(45) Date de publication du fascicule du brevet:
21.08.85

(51) Int. Cl.⁴: **C 07 C 29/54,** C 07 C 29/74,
C 07 C 31/38

(21) Numéro de dépôt: 83400268.5

(22) Date de dépôt: 08.02.83

(54) **Procédé de préparation de perfluoroalcoyl-2-éthanols.**

(30) Priorité: 23.02.82 FR 8202922

(43) Date de publication de la demande:
28.09.83 Bulletin 83/39

(45) Mention de la délivrance du brevet:
21.08.85 Bulletin 85/34

(84) Etats contractants désignés:
BE CH DE FR GB IT LI NL SE

(56) Documents cités:
DE - A - 2 318 677
DE - A - 3 016 571
FR - A - 2 373 503

TETRAHEDRON, vol. 33, no. 16, 1977, pages 2061-2067,
Pergamon Press, Oxford, GB., H. BLANCOU et al.:
"Reactivite des perfluorhalogenoalcanes en presence
de couples metalliques-II"

(73) Titulaire: **ATOCHEM, 12/16, allée des Vosges,
F-92400 Courbevoie (FR)**

(72) Inventeur: **Blancou, Hubert Jean, Le Sylvie - Bâtiment A
Rue Paul Rimbaud, F-34000 Montpellier (FR)**
Inventeur: **Benefice, Sylvie Résidence le Prieuré,
Bâtiment P-1 31 bis, avenue Saint-Lazare,
F-34000 Montpellier (FR)**
Inventeur: **Commeyras, Auguste André Aimé, 6 Impasse
des Ecoles, F-34960 Clapiers (FR)**

(74) Mandataire: **Rochet, Michel et al, ATOCHEM
Département Propriété Industrielle Cédex 22,
F-92091 Paris la Défense (FR)**

BUNDESDRUCKEREI BERLIN

## Description

La présente invention concerne un procédé quantitatif de fabrication d'alcools polyfluorés du type $R_FCH_2CH_2OH$ où $R_F$ est un radical perfluoré.

Ces alcools sont des matières premières pour la synthèse de produits chimiques à chaîne perfluorée utilisables par exemple comme agents de surface.

Ils ont été obtenus jusqu'à ce jour par action de l'iodure $R_FCH_2CH_2I$ sur un amide en solution aqueuse, comme décrit dans le brevet japonais n° 37 520/1972, ou par action du même iodure sur un oléum sulfurique, procédé divulgué par le brevet US n° 3 283 012. Cependant, ces procédés aboutissent à des rendements moyens, respectivement de l'ordre de 71 et 84% ce qui est un gros inconvénient compte-tenu du prix élevé des matières polyfluorées mises en oeuvre.

La réaction des iodures $R_FI$ sur un couple métallique tel que Zn/Cu, dans un solvant dissociant comme le DMSO ou le DMF est également connue. Cette réaction hétérogène conduit par action ultérieure du produit intermédiaire ainsi obtenu sur des substrats divers à de nombreux composés perfluorés fonctionnels.

La demanderesse a mis eu point un procédé de préparation des alcools $R_FCH_2CH_2OH$ qui comprend la réaction des iodures $R_FCH_2CH_2I$ sur le couple Zn/Cu dans un solvant dissociant constitué par un phosphat d'alcoyle ou d'aryle. Cette réaction a lieu à l'abri de l'humidité. Le produit intermédiaire obtenu est oxydé, en solution, par un courant d'oxygène gazeux, puis hydrolysé par un mélange eau/acide chlorhydrique en milieu tamponné par exemple par un tampon acide formique/formiate de sodium.

Le rendement en alcool dépand de la teneur en eau du solvant. Celle-ci peut varier de 0% à 0,4% molaires, suivant le type de solvant utilisé.

Les exemples 1 à 3 illustrent l'invention sans toutefois la limiter.

### Exemple 1

On prépare le couple métallique Zn/Cu de la manière suivante:

A une solution de 0,2 g d'acétate de cuivre (0,001 mole) dans 10 ml d'acide acétique portée à ébullition, on ajoute par petites fractions 6,5 g de zinc en poudre fine. Le couple ainsi préparé, lavé 3 fois avec 40 ml de DMSO anhydre est prêt à l'emploi.

Par ailleurs, du phosphate de butyle est déshydraté jusqu'à une teneur en eau inférieure à 0,25% molaire par séjour prolongé sur tamis moléculaire ou sur une résine desséchante.

On ajoute 0,05 mole de $C_4F_9CH_2CH_2I$, soit 18,7 g, goutte-à-guotte sur le couple Zn/Cu dispersé dans 30 ml de phosphate de butyle contenant 0,25% d'eau.

Le mélange est proté à une température de 80°C et vigoureusement agité pendant deux heures. Un courant d'oxygène est envoyé ensuite dans le mélange réactionnel durant 10 minutes, puis le mélange est tamponné par addition d'une solution acide formique/formiate de sodium et hydrolysé par une solution d'acide chlorhydrique aqueux à 20%.

Après décantation, la partie organique est extraite à léther et distillée sous une pression de 20 mm Hg.

On obtient 13,2 g d'un de point d'ébullition 65°C sous 20 mm Hg, identifié comme $C_4F_9CH_2CH_2OH$, ce qui correspond à un rendement de 100%.

### Exemple 2

On opère comme dans l'exemple 1, mais avec 0,05 mole de $C_6F_{13}CH_2CH_2I$, soit 23,7 g. On obtient 18,2 g d'un produit de point d'ébullition 87°C sous 20 mm Hg, ce qui correspond à un rendement de 100% de $C_6F_{13}CH_2CH_2OH$.

### Exemple 3

On opère comme dans l'exemple 1, mais avec 0,05 mole de $C_8F_{17}CH_2CH_2I$, soit 28,7 g. On obtient 23,2 g d'un produit de point d'ébullition 115°C sous 20 mm Hg ce qui correspond à un rendement de 100% de $C_8F_{17}CH_2CH_2OH$.

Les exemples 4 à 8 suivants sont donnes à titre comparatif.

### Exemples 4 et 5

On opère comme dans les exemples 1 et 2 mais en utilisant comme solvant du DMSO contenant 0,2% molaire d'eau.

Les rendements sont les suivants:

avec $R_F = C_4F_9$     Rendement = 10%
      $R_F = C_6F_{13}$     Rendement = 10%

### Exemples 6 et 7

On opère comme dans les exemples 1 et 2 mais en utilisant comme solvant du DMF contenant 0,2% molaire d'eau.

Les rendements sont les suivants:

avec $R_F = C_4F_9$     Rendement = 25%
      $R_F = C_6F_{13}$     Rendement = 25%

### Exemple 8

On opère comme dans l'exemple 1 mais en utilisant comme solvant du carbonate d'éthyle à 0,2% molaire d'eau.

On obtient 30% d'un alcool avec $R_F = C_4F_9$.

## Revendications

1. Procédé de préparation d'alcools poly-fluorés du type $R_FCH_2CH_2OH$ où $R_F$ est un radical perfluoré, caractérisé en ce que l'on fait réagir l'iodure $R_FCH_2CH_2I$ sur le couple Zn/Cu dans un phosphate d'alcoyle ou d'aryle ayant une teneur en eau inférieure à 0,4% molaire, puis soumet le mélange à une oxydation par un courant d'oxygène gazeux et à une hydrolyse acide en milieu tamponné.

2. Procédé selon la revendication 1 dans lequel phosphate d'alcoyle ou d'aryle présente une teneur en eau inférieure à 0,25% molaire.

3. Procédé selon la revendication 1 ou 2 dans lequel on emploie le phosphate de butyle.

## Patentansprüche

1. Verfahren zur Herstellung von polyfluorierten Alkoholen des Typs $R_FCH_2CH_2OH$, wobei $R_F$ einen perfluorierten Rest bedeutet, dadurch gekennzeichnet, daß man das Jodid $R_FCH_2CH_2I$ auf das Paar Zn/Cu in einem Alkyl- oder Arylphosphat mit einem Wassergehalt von weniger als 0,4 Mol-% einwirken läßt, und dann die Mischung einer Oxydation durch einen Sauerstoffstrom und einer sauren Hydrolyse in gepuffertem Milieu unterwirft.

2. Verfahren nach Anspruch 1, in dem das Alkyl- oder Arylphosphat einen Wassergehalt von weniger als 0,25 Mol-% aufweist.

3. Verfahren nach den Ansprüchen 1 oder 2, in dem Butylphosphat eingesetzt wird.

## Claims

1. Process for the preparation of polyfluorinat-ed alcohols of the $R_FCH_2CH_2OH$ type, where $R_F$ is a perfluorinated radical, characterised in that the iodide $R_FCH_2CH_2I$ is reacted with the Zn/Cu couple in an alkyl phosphate or aryl phosphate having a water content of less than 0.4 mole %, after which the mixture is subjected to oxidation with a stream of oxygen gas and to acid hydrolysis in a buffered medium.

2. Process according to Claim 1, in which the alkyl phosphate or aryl phosphate has a water content of less than 0.25 mole %.

3. Process according to Claim 1 or 2, in which butyl phosphate is employed.